# EUROPEAN PATENT SPECIFICATION

(11) **EP 2 452 640 B1**
(45) Date of publication and mention of the grant of the patent: **08.11.2017**
(21) Application number: 12152595.0
(22) Date of filing: 16.04.2008
(51) Int. Cl.: A61B 17/34, A61M 39/10, A61M 39/12

(54) **Adapter for an introducer**
Adapter für einen inserter
Adaptateur pour introducteur

(30) Priority: 20.04.2007 US 738023
(43) Date of publication of application: 16.05.2012
(62) Divisional of application: 08737452.6
(73) Proprietor: St. Jude Medical Coordination Center BVBA, 1930 Zaventem (BE)
(72) Inventor: Preinitz, Fredrik, 753 50 UPPSALA (SE)
(74) Representative: Brann AB

(56) References cited:
- GB-A- 505 212
- US-A- 1 558 829
- US-A- 2 423 762
- US-A- 4 511 163
- US-A1- 2002 069 879

## Description

### Field of the Invention

The present invention relates generally to an introducer adapter for an introducer which through a percutaneous puncture is used to obtain access to blood vessels, cavities, organs or other bodily tissues in order to perform a medical operation, and more particularly to an adapter being provided with a tapered externally threaded end, which is screwed or inserted into an introducer already in place at the puncture site.

### Background of the Invention

An introducer is an elongated tubular member, which in the field of medical surgery is used to gain access to a particular site within a patient's body. It should be noted that the term introducer is, unless otherwise indicated, used herein as indicative of an introducer sheath which comprises at least one proximal entry port. Depending on the medical procedure, the design of an introducer can vary. For example, the proximal end can comprise an attachment means for a medical instrument. Additionally, an entry port can comprise sealing material to prevent leakage of e.g. bodily fluids. Furthermore, an introducer can comprise several entry ports. Various examples of attachment means have been described. US Patent 2,423,762 discloses a liquid-tight joint for a hypodermic syringe comprising a conical member provided with a continuous screw thread on its outside. GB Patent Application 505 212 also relates to hypodermic syringes and the overall object of the device is to achieve a liquid-tight mounting for attachment of a conical nozzle. US Patent Application 2002/069879 is an example of a similar arrangement using a connection adapter in a retainer for a laryngeal mask; however, here the purpose is, to allow air to flow also through the connection site. Usually the medical operation wherein an introducer is used commences with a puncture operation in which a hollow needle is introduced at a point on a patient's skin, and is then advanced through tissues beneath the skin to the position in the organ of interest, e.g. a blood vessel. A guide wire is then introduced through the needle, whereupon the needle is removed, and an introducer together with a dilator is advanced over the guide wire. After removal of the guide wire and the dilator, access to the organ has now been obtained through the lumen of the tubular introducer.

The procedure described above can be used to gain access to a blood vessel for performing different types of intravascular operations. When the operation in question is completed, the puncture hole in the blood vessel wall can be sealed by a closure device, wherein an inner member of said closure device is positioned at an inner surface of the vessel wall by means of an insertion device and which is held in place by a suture or filament. The suture or filament extends from the closure device, through tissue overlaying the vessel, and out of the skin surface. An example of this technique is disclosed in U.S. Patent 4,744,364.

In many cases several different medical procedures need to be performed on a patient using the same access site, such as a percutaneous blood vessel puncture. Some examples of different procedures involving such a puncture are insertion of and measurements using a sensor guide wire, placing a stent, performing angioplasty and, in most cases, sealing the puncture, as described above. Especially in the case of sealing the puncture, which follows after one or several different medical procedures, a need arises to switch introducer sheaths, due to specific requirements on the access path of the closure device.

### Summary of the Invention

As mentioned above, introducers are of many different dimensions and/or designs. Although the introducers in prior art serve their intended purposes, changing the introducers increases the risk of causing infection, excessive bleeding due to ruptures of blood vessels, and discomfort or pain to the patient. Prior art shows several examples of adapters to be able to connect to an introducer. Two such examples are described in EP 1305076 and US 5,147,336. However, these adapters require a specific shape or dimension of a matching mating member on the introducer. Therefore there is a need for a way to use an introducer which is already in place in e.g. a blood vessel for a subsequent medical procedure, irrespective of the shape and dimensions of the introducer. The present invention is defined in claim 1 while preferred embodiments are set forth in the dependent claims. The present invention provides an introducer adapter which can be inserted into a pre-placed introducer of arbitrary dimensions, eliminating the need to replace the introducer. The invention provides an introducer adapter which can be used to connect any pre-placed introducer with any new introducer, without having to remove the first introducer. The introducer adapter may comprise, not according to the invention, an insertion tool for a closure device.

According to one embodiment of the present invention a universal introducer adapter comprises a tapered externally threaded section which is attached to the pre-placed introducer by screwing the adapter directly into the introducer sheath. The continuously tapered shape allows the attachment to introducers with a wide range of inner dimensions. The threads provide means to firmly attach the introducer adapter and seal the passageway from leakage of e.g. blood or other bodily fluids. Using the adapter eliminates the need for replacement of the introducer when switching medical procedures requiring introducer sheaths with different dimensions and/or different configurations. According to one embodiment the adapter with a tapered externally threaded end is the distal end of a new introducer, needed for a subsequent medical procedure. The adapter may be, not according to the invention, an integrated part of a medical device, such as an insertion tool for a closure device. In a further embodiment, the distal end of the tapered threaded section comprises an extended tubular section, which reaches beyond the distal end of the pre-placed introducer.

### Brief Description of the Drawings

Figs. 1 a and 1b illustrate the present invention according to a first embodiment.
Fig. 2 illustrates the present invention according to a second embodiment.
Fig. 3 illustrates the present invention according to a third embodiment.
Fig. 4 illustrates the present invention according to a fourth embodiment.
Fig. 5 illustrates the present invention according to a fifth embodiment.
Fig. 6 illustrates the present invention according to a sixth embodiment.

### Detailed Description of Preferred Embodiments

The present invention will be described in the context of a percutaneous puncture made to gain access to a blood vessel. However, the present invention can be also used in other procedures, such as obtaining access to the abdominal cavity or a specific organ through an introducer.

From prior art, it is known to provide a connecting device for a tubular device by providing an adapter with a tapered shape. One such example is described in U.S. Patent 5,776,117, which describes an adapter for connection to various sized tubes, adapters and/or y-ports provided with several sections with different external diameters, together forming a roughly tapered shape, with a stepwise changing diameter. In addition, the adapter described in U.S. Patent 5,776,117 preferably requires an elastic tube, and is not truly universal in the sense of being able to connect to any commonly used dimension or type of tube. To be truly universal, an adapter needs to be able to attach to all possible dimensions of tube or introducer. In the present invention, this has been solved by providing a continuously tapered gripping surface, with cross-sectional diameters covering the range of commonly used inner diameters in commercially available introducers.

Furthermore, when connecting an introducer adapter to a percutaneous introducer, especially one used to gain access to a blood vessel, it is important that the introducer which is already in place at the access site not be moved more than absolutely necessary, as this can cause leaking of bodily fluids and tissue damage. Using an adapter designed to attach by friction only requires extensive pressure to attach the adaptor to a tube or introducer, and thereby greatly increases the risk of moving the pre-placed introducer too much. The present inventor has realized that using threads, in particular penetrating or cutting threads, greatly eases the procedure of attaching the introducer adapter to a pre-placed introducer without risking tissue damage or dislodging the pre-placed introducer.

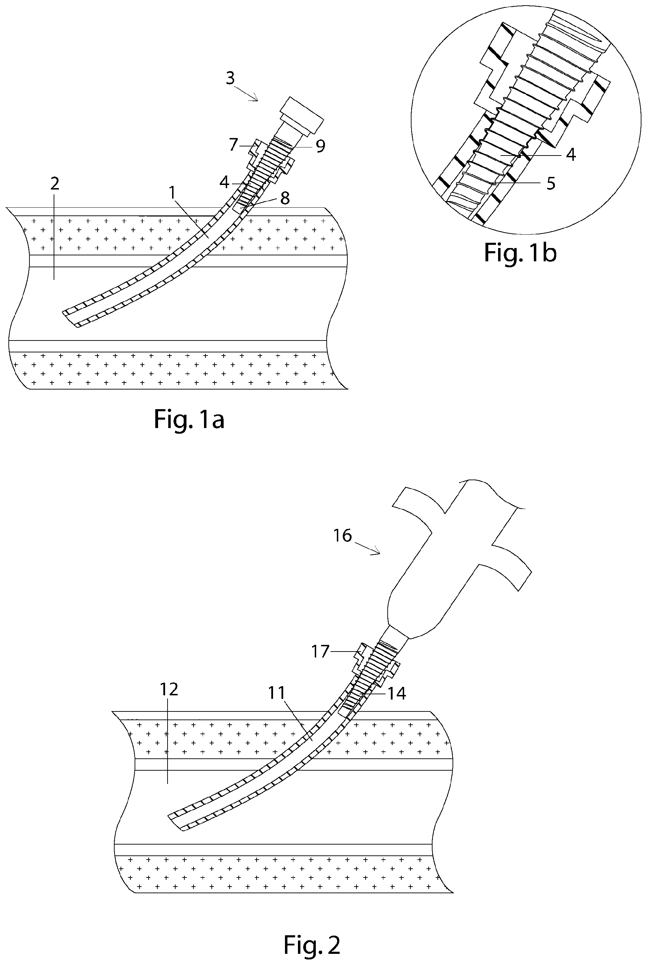

A first embodiment of the present invention is illustrated in Figs. 1 a and 1b. A pre-placed introducer 1 is present in a percutaneous puncture into e.g. a blood vessel 2. This introducer can have been used for a variety of purposes, such as insertion of a guide wire, the drawing of a blood sample or infusion of a solution into the blood stream. Depending on the purpose and the retailer of said introducer, it can have a variety of dimensions and shapes. The introducer 1 in Fig. 1 a is a simple schematic of such an introducer. Normally the inner dimension of an introducer is between 3 French and 10 French (approximately 1.0 mm and 3.3 mm, respectively). In this embodiment, the present invention comprises a new introducer 3, necessary for a subsequent procedure, comprising a tapered distal section 4 provided with a plurality of external threads 5. This is illustrated in Fig. 1 a with a magnification of the tapered section in Fig. 1 b. Introducer 3 is provided with an inner lumen extending from the proximal end to the distal end, providing access to the inner lumen of the pre-placed introducer 1, and thereby also to the lumen of blood vessel 2, through introducer 3.

The following is described in reference to Figs. 1 a and 1b, but is also applicable to the embodiments described in Figs. 2-4. The distal tapered section 4 is attached by screwing introducer 3 into the pre-placed introducer 1 such that at least one full turn or rotation of the threads grips the inner wall of the pre-placed introducer 1 (see magnification in Fig. 1b). The threads are preferably designed to partly penetrate into or deform the pre-placed introducer. This produces a secure tight seal between the pre-placed introducer and the present invention, in order to minimize loss of bodily fluids such as blood. In addition, this ensures a safe and rapid attachment procedure, necessitating less force and risk of moving the pre-placed introducer. Due to the continuously tapered shape of the tapered section 4, the present invention can attach to a range of different inner dimensions of the pre-placed introducer 1. In some embodiments, the tapered distal section 4 is preferably designed such that the smallest diameter of the section provided with threads is approximately 3 French and the largest diameter provided with threads is approximately 10 French. In some embodiments, the angle of the taper (that is the angle between the surface from which the threads project and the centerline of the adapter) is less than 30 degrees, preferably less than 20 degrees, preferably between 5 and 20 degrees. Using these dimensions ensures that the introducer adapter is truly universal in use, i.e. can be used for all commercially available introducers.

Furthermore, it is also within the scope of the present invention that the threads attach to a more proximal part of the pre-placed introducer, such as a softer seal in a hub 7 of the introducer 1, often provided to prevent blood loss when accessing a blood vessel.

As described earlier herein, the term introducer comprises an introducer sheath and, if present, an integrated cap, valve or hub. The following is described in reference to Figs. 1 a and 1b, but is also applicable to the embodiments described in Figs. 2-6. To be able to attach to the inner walls of a pre-placed introducer, the maximum external cross-sectional dimension of the narrowest part 8 of the tapered section 4 is smaller than a proximal inner dimension of the pre-placed introducer, and the external cross-sectional dimension of a widest part 9 of the tapered section 4 exceeds that of a proximal inner dimension of the pre-placed introducer. Thereby the present invention, when screwed or inserted into a pre-placed introducer, will grip and seal tight along an arbitrary section of the threads of the tapered section and the inside of the pre-placed introducer. This is illustrated in Figures.

It should be noted that it is also within the scope of the present invention that the tapered section, as mentioned above, grips and seals tight against a section inside the cap, valve or hub of the pre-placed introducer. In the latter case, this can provide the advantage of not decreasing the diameter of the available inner lumen of the introducer when attached to the adapter. Furthermore, in this context, it should also be noted that it is within the scope of the present invention that the abovementioned inner diameters of the pre-placed introducer allow for resilience by employing a soft or pliable material. A non-limiting example of such a situation is when a soft sealing material, such as silicone or polyurethane, is provided as a gasket in an introducer's hub, which is displaced by insertion of the adapter of the present invention. However, it should be noted that due to the cutting or penetrating gripping means of the introducer adapter, there is no need for elastic or soft material in the pre-placed introducer in order for the tapered distal section to attach to the pre-placed introducer, in fact, the introducer will attach well to the common hard plastics in the elongated tubular part of commercially available introducers.

In a second embodiment, illustrated in Fig. 2, a tapered distal section 14 is an integral part of a medical device 16, such as one used to insert an instrument or perform closure of the percutaneous puncture. Also in this embodiment, the tapered section is provided with a plurality of external threads (not labeled in Fig. 2, refer to Fig. 1b). Fig. 2 also illustrates a hub 17.

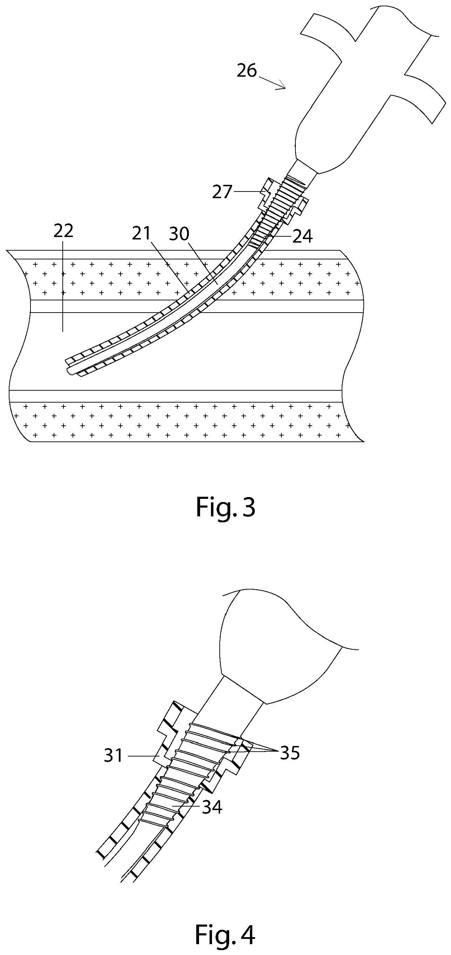

In a further embodiment, a distal end 30 of the present invention is extended such that the tip reaches beyond the distal end of a pre-placed introducer 21. This is illustrated in Fig. 3. When e.g. performing a closure procedure of the percutaneous puncture, it is advantageous if the dimensions of the entire access route of sealing discs are compatible with the medical device used. By extending the distal end, the delivery of e.g. a sealing disc is not compromised due to a change in size and/or shape of the delivery channel while transporting the disc from the medical device into the blood vessel. It should be noted that in this embodiment the extended section 30, distal to the tapered section 24, is preferably made of a material that is soft enough to follow the shape of the pre-placed introducer, without inhibiting the manipulation of the introducer.

Similar to the embodiments in Figs. 1a, 1b and 2, the third embodiment in Fig. 3 is provided with an inner lumen extending from the distal end, which, when the device is placed in a blood vessel 22, provides access to the lumen of said blood vessel 22, up into the interior of a medical device 26. Fig. 3 also illustrates a hub 27.

In Figs. 1-3, the threads are illustrated as approximately 18 rotations or turns around the tapered section 4, 14, 24. As used herein, the term "threads" encompasses the type of arrangement shown in Fig. 1 b. That is, Fig. 1b shows "threads" even though there is only one continuous projection forming item 5 in Fig. 1 b. It should be noted that it is within the scope of the present invention that the provided number of rotations of the threads around the tapered section is more than one full turn, to provide good sealing properties. However, to achieve a wide range of use for the adapter, it can be provided with up to 100 rotations or more. In addition, the threads can be provided as several parallel threads. This is illustrated in Fig. 4, where three parallel threads 35 extend in a spiral or coil along the tapered section 34. Due to the steep turn in such an arrangement, the tapered section 34 obtains faster gripping action (i.e. necessitating less effort) when screwing the adapter into the pre-placed introducer. It should be noted that Fig. 4 illustrates a non-limiting example, and that the number of parallel threads 35 can also be two, four or more.

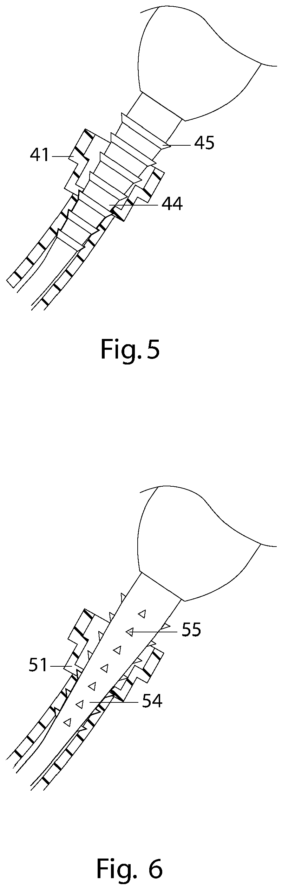

Yet another embodiment of the present invention is illustrated in Fig. 5. Here a tapered section 44 is provided with threads or ridges 45 that are perpendicular to the central axis of the tapered section. The ridges 45 thereby form circumferential protrusions. Furthermore, the outer edges of the ridges are preferably angled, such that when the adapter is inserted or screwed into the pre-placed introducer, the ridges will allow easy insertion, while providing resistance when pulling back on the adapter. In a further embodiment, illustrated in Fig. 6, the ridges are replaced by several barbs or hooks 55, which are randomly or systematically positioned on the surface of the tapered section 54. These act as fasteners when the adapter is pulled back, such that the adapter is fitted within the introducer.

The material of the continuously tapered section 4, 14, 24, 34, if provided with threads, should preferably be harder than that of the walls of the pre-placed introducer 1, 11, 21, 31 such that the threads will seal tightly against the inner wall, by slightly deforming or cutting into the inner wall (see Figs. 1-4). Similarly, the tapered section, if provided with ridges or barbs 44, 54, as illustrated in Figs. 5-6, can be of a harder material such that the ridges or barbs will penetrate or displace the walls of the pre-placed introducer 41, 51. Non-limiting examples of such materials are stainless steal, other metals, polycarbonate, polysulfonate, polyetheresterketone, other polymers, or carbon fiber composites. However, it should be noted that e.g. in the embodiment illustrated in Fig. 5, the threads or ridges 45 can comprise a softer material and the tapered section 44 a harder material, which also provides tight sealing action by the ridges against the inner wall of the pre-placed introducer. Similarly, in the embodiment in Fig. 6, the barbs 55 can be made of a softer material, providing resistance when retracting the adapter.

Although the present invention has been described with reference to specific embodiments it will be apparent to those skilled in the art that many variations and modifications can be performed. For example, the adapter can be an integral part of an insertion tool for a closure device comprising an inner sealing disc and an outer sealing disc. Furthermore, the adapter can be an integral part of an insertion tool for a closure device comprising an inner anchor member and an outer sealing member, e.g. a collagen plug. In addition, the adapter can be provided as a separate member to be used together with insertion tools such as the two abovementioned tools. It should be noted that the term "gripping means" or "gripping structure" as used herein comprises threads, ridges, hooks, barbs or other protruding members that can act as fasteners to firmly hold the adapter positioned within an introducer. The embodiments described above can also be used in conjunction with the techniques and devices described in U.S. Patent No. 7,073,509, US Patent No. 7,094,209 and U.S. Patent Application Publication No. 2004/0093025. All three of these documents are assigned to the assignee of this patent specification.

## Claims

1. An introducer adapter (3) for connecting a medical device to a first pre-placed percutaneous introducer, wherein said introducer adapter is a universal introducer adapter provided with an inner lumen extending from the proximal end to the distal end, **characterised in that** said introducer adapter (3) comprises a tapered distal section (4, 14, 24, 34, 44, 54) provided with external gripping and cutting or penetrating means, wherein said introducer adapter (3) is separate from said medical device.

2. An introducer adapter (3) according to claim 1, wherein said gripping means comprises threads.

3. An introducer adapter (3) according to claim 1, wherein said gripping means are chosen from the group comprising ridges, circumferential protrusions, hooks and barbs.

4. introducer adapter (3) according to claim 2, wherein the tapered distal section is designed such that the smallest diameter of the section provided with threads is approximately 1.0 mm and the largest diameter provided with threads is approximately 3.3 mm.

5. An introducer adapter (3) according to claim 2, wherein the angle of the taper, being the angle between the surface from which the threads project and the centerline of the adapter, is less than 30 degrees.

6. An introducer adapter (3) according to claim 2, wherein said threads comprise two or more parallel threads.

7. An introducer adapter (3) according to claim 1, wherein said tapered distal section is made of stainless steel, polycarbonate, polysulfonate, polyetheresterketone, or carbon fiber composites.

## Patentansprüche

1. Einführhilfenadapter (3) zum Verbinden einer medizinischen Vorrichtung mit einer ersten, vorher angeordneten perkutanen Einführhilfe, wobei der Einführhilfenadapter ein Universaleinführhilfenadapter ist, der mit einem inneren Lumen versehen ist, das sich von dem proximalen Ende zu dem distalen Ende erstreckt, **dadurch gekennzeichnet, dass** der Einführhilfenadapter (3) einen konisch zulaufenden distalen Abschnitt (4, 14, 24, 34, 44, 54) umfasst, der mit äußeren Greif- und Schneide- oder Penetrationsmitteln versehen ist, wobei der Einführhilfenadapter (3) von der medizinischen Vorrichtung getrennt ist.

2. Einführhilfenadapter (3) nach Anspruch 1, wobei die Greifvorrichtung Gewindegänge umfasst.

3. Einführhilfenadapter (3) nach Anspruch 1, wobei die Greifvorrichtungen aus der Gruppe, umfassend Rippen, umlaufende Vorsprünge, Haken und Widerhaken, ausgewählt sind.

4. Einführhilfenadapter (3) nach Anspruch 2, wobei der konisch zulaufende distale Abschnitt so ausgelegt ist, dass der kleinste Durchmesser des mit Gewindegängen versehenen Abschnitts etwa 1,0 mm beträgt und der größte mit Gewindegängen versehene Durchmesser etwa 3,3 mm beträgt.

5. Einführhilfenadapter (3) nach Anspruch 2, wobei der Winkel des Konus, bei dem es sich um den Winkel zwischen der Oberfläche, von der die Gewindegänge vorragen, und der Mittellinie des Adapters handelt, weniger als 30 Grad beträgt.

6. Einführhilfenadapter (3) nach Anspruch 2, wobei die Gewindegänge zwei oder mehr parallele Gewindegänge umfassen.

7. Einführhilfenadapter (3) nach Anspruch 1, wobei der konisch zulaufende distale Abschnitt aus Edelstahl, Polycarbonat, Polysulfonat, Polyetheresterketon oder Kohlefaserverbundstoffen hergestellt ist.

## Revendications

1. Adaptateur pour introducteur (3) destiné à raccorder un dispositif médical à un premier introducteur percutané pré-positionné, dans lequel ledit adaptateur pour introducteur est un adaptateur pour introducteur universel pourvu d'un lumen interne s'étendant de l'extrémité proximale à l'extrémité distale, caractérisé en ce ledit adaptateur pour introducteur (3) comprend une section distale conique (4, 14, 24, 34, 44, 54) pourvue de moyens externes de saisie et de découpe ou de pénétration, dans lequel ledit adaptateur pour introducteur (3) est séparé dudit dispositif médical.

2. Adaptateur pour introducteur (3) selon la revendication 1, dans lequel lesdits moyens de saisie comprennent des fils.

3. Adaptateur pour introducteur (3) selon la revendication 1, dans lequel lesdits moyens de saisie sont sélectionnés dans le groupe comprenant des stries, des saillies circonférentielles, des crochets et des pointes.

4. Adaptateur pour introducteur (3) selon la revendication 2, dans lequel la section distale conique est conçue de sorte que le plus petit diamètre de la section pourvu de fils soit approximativement 1,0 mm et que le plus grand diamètre pourvu de fils soit approximativement 3,3 mm.

5. Adaptateur pour introducteur (3) selon la revendication 2, dans lequel l'angle du cône, qui est l'angle entre la surface à partir de laquelle les fils font saillie et la ligne médiane de l'adaptateur, est inférieur à 30 degrés.

6. Adaptateur pour introducteur (3) selon la revendication 2, dans lequel lesdits fils comprennent deux fils parallèles ou plus.

7. Adaptateur pour introducteur (3) selon la revendication 1, dans lequel ladite section distale conique est réalisée en acier inoxydable, en polycarbonate, en polysulfonate, en polyéther-ester-cétone, ou en composites en fibre de carbone.
